# EUROPEAN PATENT SPECIFICATION

(11) **EP 3 378 498 B1**
(45) Date of publication and mention of the grant of the patent: **22.07.2020**
(21) Application number: 18162960.1
(22) Date of filing: 20.03.2018
(51) Int. Cl.: A61L 2/10, B60N 3/02

(54) **SANITISING SYSTEM FOR PUBLIC USE SUPPORT COLUMNS**
DESINFEKTIONSSYSTEM FÜR STÜTZSÄULEN FÜR DEN ÖFFENTLICHEN GEBRAUCH
SYSTÈME D'ASSAINISSEMENT DES COLONNES DE SUPPORT POUR UNE UTILISATION PUBLIQUE

(30) Priority: 20.03.2017 IT 201700030525
(43) Date of publication of application: 26.09.2018
(73) Proprietor: IVECO FRANCE S.A.S., 69200 Vénissieux (FR)
(72) Inventor: CODRON, Stephane, 69008 LYON (FR); BONNAUD, Bernard, F-79440 COURLAY (FR)
(74) Representative: Bongiovanni, Simone

(56) References cited:
- WO-A1-2009/072121
- CN-U- 204 586 610
- JP-U- S6 386 234
- US-A1- 2014 264 076

## Description

### PRIORITY CLAIM

The present invention relates to a system for sanitising support columns for public use, in particular a system for sanitising columns of a public passenger transport vehicle, for example a bus.

Support columns are normally used in several types of vehicles, for example in public passenger transport road vehicles. These columns are needed for passengers to support themselves so that the passengers do not fall over during changes in the vehicle's acceleration.

However, given the large number of passengers who may travel on a vehicle in a day, the above-mentioned columns are obviously contaminated by the passengers.

In fact, by holding onto or sneezing in the vicinity of the column, the passengers can deposit on the columns germs (for example viruses and/or bacteria) which could easily be transmitted to other passengers who touch the same contaminated portion of the column.

In order to ensure an acceptable level of hygiene, said columns are cleaned with specialised chemical products on a weekly frequency defined by the operator.

It takes a long time to clean all of a vehicle's columns at the end of the vehicle's service turn. It is also not possible to clean the columns several times a day because this would cause the vehicle to be out of service for too long with a consequent increase in costs for the public transport companies responsible for providing the service.

In addition, the chemical products used to clean the columns are dangerous for the staff and the environment.

Examples of cleaning devices are disclosed in documents WO2009072121 A1, US2014264076 A1, CN204586610 U or JPS6386234 U.

It is therefore still necessary to increase the possibility of cleaning the vehicle quickly, cost-effectively, and in way that safeguards staff and is environmentally friendly.

The objects of this invention are to resolve the above-mentioned problems.

The above-mentioned objects are achieved by a system for sanitising columns for public use according to claim 1.

Other characteristics and advantages of the invention will become apparent from the following non-limiting description, provided for illustrative purposes, with reference to the attached drawings in which:
- Figure 1 is a partially sectional side view of a public passenger transport vehicle comprising a system according to the present invention;

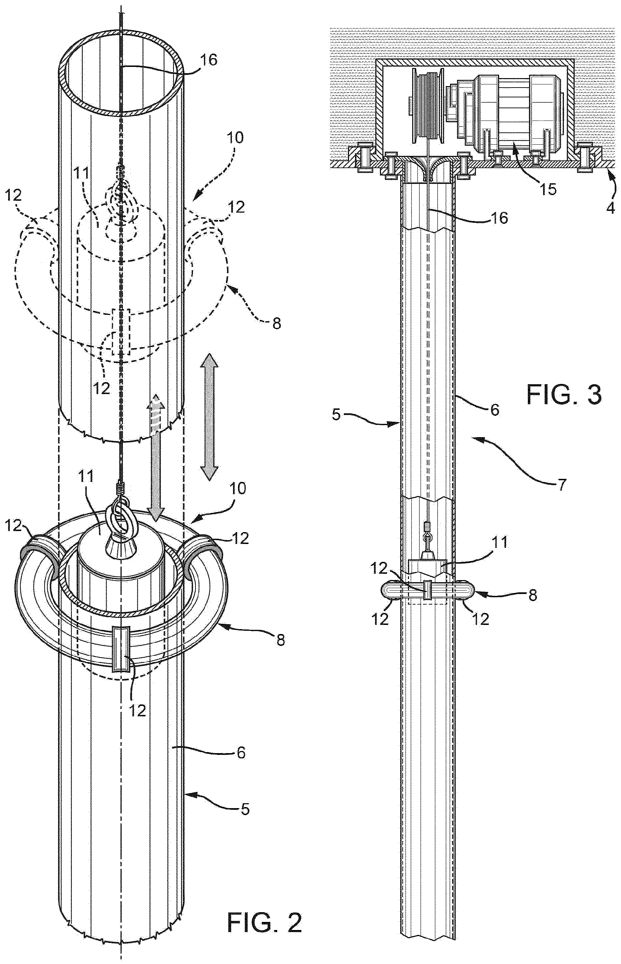

- Figure 2 is a sectional side view of a system according to the present invention; and
- Figure 3 is a perspective view of the operation of the system of Figure 2.

The present description relates to a public passenger transport vehicle, a bus, without however any loss of generality.

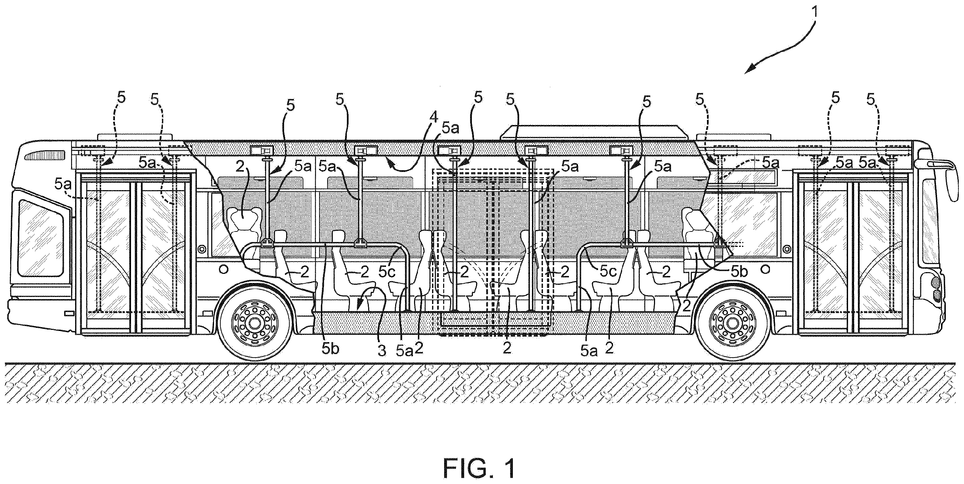

Figure 1 illustrates a bus 1 of known type, essentially comprising a plurality of seats 2 positioned in the interior of the bus 1.

In a known way and according to the distribution of seats, the bus 1 comprises a plurality of columns 5 configured to allow passengers to support themselves during their journey aboard the bus 1 and fixed to the floor 3 and/or the ceiling 4 of the bus.

The columns are preferably either vertical columns 5a, horizontal columns 5b, or fittings and/or inclined columns 5c. The columns 5 may be individual columns like those normally provided opposite the entrance of the bus 1 or interconnected columns 5, such as those normally provided near the seats 2. In a known way, the columns 5 are made from hollow tubes.

For each uninterrupted portion of the columns 5 above, the bus 1 is equipped with a sanitising system 7 configured to sanitise a predefined part of a column by moving over said portion.

The sanitising system 7 essentially comprises an electromagnetic radiation source 8 configured to slide automatically over the column 5 thanks to operating means.

The electromagnetic radiation source 8 is configured to provide sterilising electromagnetic radiation, preferably UV-C radiation having a wavelength of between 250 nm and 100 nm. The electromagnetic radiation source 8 is preferably a neon light.

The electromagnetic radiation source 8 beneficially has an annular shape and is configured to completely surround an external surface 5a of the column 5. The electromagnetic radiation source 8 is preferably toroidal, having an inside diameter greater than the radius of the external surface 5a.

The operating means beneficially comprise a magnetic operating system 10.

The magnetic operating system 10 comprises a magnet 11 positioned inside the hollow space 5b inside the column 5 and configured to move along the column 5, as well as a plurality of metal rings 12 configured to be secured to the electromagnetic radiation source 8. The columns 5 are made from material which does not interfere with the electromagnetic interaction between the magnet 11 and the rings 12.

The rings 12 are magnetised in order to interact magnetically with the magnet 11 and thus be attracted and/or repelled by it. As a result, the electromagnetic radiation source 8, which is attached to the rings 12, follows their movements.

The rings 12 are positioned at the same angular distance from each other in order to optimise their interaction with the magnetic field of the magnet 11. Even more preferably, there are three rings 12, positioned at 120° to each other.

The magnet 11 is operated inside the column 5 by means dedicated operating means, for example by an electric motor 15 which coils and uncoils a cable 16 to which the magnet 11 is linked. The electric motor 15 is preferably located in the ceiling 4 of the bus 1 or else within the same column 5.

The sliding of the sanitising system 7 beneficially does not rub on the external surface 5a of the column 5; there is thus a small gap between the rings 12 and the external surface of the column 5. Alternatively, the rings 12 may rub slightly on the column 5 in order to guide the movement of the light source without however impeding it. In this case, the rings 12 are in direct contact with the external surface of the column 5.

The electrical supply for the electromagnetic radiation source 8 may be provided in a known manner, for example by a wire (not shown) configured to follow the movement of the radiation source, or by batteries (not shown) mounted on the radiation source.

The sanitising system 7 also comprises a device (not shown) configured to activate the electromagnetic radiation source and the operating means. For example, the control device may be an electronic unit of known type configured to communicate with the ECU of the vehicle or the control device could be the ECU itself.

Beneficially, the control device may be activated by the driver of the bus 1 or automatically based on a predefined algorithm, for example a predefined schedule.

Furthermore, the bus 1 may comprise sensor means (not shown), for example cameras, configured to detect the presence of a person supported by the column 5. The control logic could be programmed so that when the control device detects by means of the sensor means that a person has stopped supporting themselves by the column 5, it immediately actives the sanitising system 7 to sanitise the column 5.

Operation of the system to sanitise the support columns for public use is the following.

In an inactive phase, the electromagnetic radiation source 8 is positioned near the ceiling 4 of the bus 1 to prevent the passengers from touching it.

According to the predetermined logic, for example at the end of the journey of the bus 1, the control device activates the electric motor 15 which lowers the magnet 11 inside the column 5 along the whole of the portion of column 5 to be cleaned.

The electromagnetic radiation source 8, activated by the control device, is thus operated by the electromagnetic interaction between the magnet 11 and rings 12 and slides over the portion of the column 5, sanitising it.

The sliding speed is determined by the electromagnetic radiation source 8 and can be controlled and varied by the control device.

Once all of the portion of the column 5 has been sanitised, the motor 15 returns the magnet 11 to its resting position near the ceiling of the bus 1.

From the foregoing, the benefits of a system for sanitising support columns for public use according to the present invention are apparent.

The sanitising system 7 improves the hygiene of the support columns 5 of the bus 1 in a simple and rapid way without the use of chemicals.

The sanitising can also be repeated several times a day according to a predetermined algorithm without incurring downtimes for the bus 1.

Finally, it is apparent that the system for sanitising support columns for public use according to the present invention may be subject to modifications and variations.

For example, the operating system 10 could be mechanical or pneumatic, or the electromagnetic radiation source could be achieved by other than a neon light.

In addition, the sanitising system 7 has been described for a vertical column 5, whereas it could also be applied to a horizontal or oblique column, by modifying the operating system 10, in an obvious way.

## Claims

1. A system (7) for sanitising a column (5) for public use, **characterised in that** it comprises a sanitising electromagnetic radiation source (8) having an annular shape, said radiation source (8) being positionable around said column (5) to slide thereon, said system (7) comprising operating means (10) configured to operate said radiation source (8) automatically along said column (5) in order to sanitise a pre-established part thereof, wherein said operating means (10) comprise a magnet (11) configured to slide inside said column (5), said radiation source (8) comprising a plurality of metal rings (12) secured to said radiation source (8) and equally angularly spaced one with respect to the other, said rings (12) being magnetised to magnetically interact with said magnet (11) so that a movement of the magnet (11) inside said column (5) is followed by an equal movement of said radiation source (8).

2. The system according to claim 1, wherein said magnet is operated by an electric motor (15) configured to be fixed with respect to said column (5).

3. The system according to claim 2, wherein said electric motor (15) is positionable inside said column (5).

4. The system according to one of the previous claims, wherein said electromagnetic radiation source (8) is toroidal.

5. The system according to one of the previous claims wherein said electromagnetic radiation source (8) is a UV-C neon light.

6. The system according to one of the previous claims, wherein the power supply of said electromagnetic radiation source (8) is a battery, said battery being mounted on said electromagnetic radiation source (8), or supported by means of a cable, said cable following the movement of said electromagnetic radiation source (8).

7. The system according to one of the previous claims, wherein the activation of said means and of said electromagnetic radiation source (8) is either manual or automatic based on a predetermined logic.

8. A vehicle (1) comprising at least one column (5) fixed with respect to said floor (3) of said vehicle (1), said column (5) being configured to allow the passengers to support themselves by said column, said vehicle (1) comprising a system (7) for sanitising at least one said column (5) according to any of the claims 1 to 7.

9. Vehicle according to claim 8, wherein said column (5) is a vertical, horizontal, or inclined column (5) with respect to the floor (3) of said vehicle (1).

10. Use of a system (7) according to any of the claims 1 to 7 for sanitizing a column (5) of a public transport vehicle.

11. Use of a system according to claim 10, wherein said electromagnetic radiation source (8) slides on said column (5), said sliding being frictionless with respect to said column (5).

## Patentansprüche

1. Ein System (7) zur Desinfektion einer Säule (5) für die öffentliche Nutzung, **dadurch gekennzeichnet, dass** es eine ringförmige desinfizierende elektromagnetische Strahlungsquelle (8) umfasst, wobei die Strahlungsquelle (8) um die Säule (5) herum angeordnet werden kann, um darauf zu gleiten, wobei das System (7) Betriebsmittel (10) umfasst, die ausgeführt sind, um die Strahlungsquelle (8) automatisch entlang der Säule (5) zu betreiben, um einen vorgegeben Teil davon zu desinfizieren,
wobei die Betriebsmittel (10) einen Magneten (11) umfassen, der ausgeführt ist, um innerhalb der Säule (5) entlang zu gleiten, und die Strahlungsquelle (8) eine Mehrzahl an Metallringen (12) umfasst, die an der Strahlungsquelle (8) befestigt sind und in gleichen Winkelabständen zueinander verteilt sind, wobei die Ringe (12) magnetisiert sind, um magnetisch mit dem Magneten (11) zu interagieren, sodass eine Bewegung des Magneten (11) innerhalb der Säule (5) von einer gleichartigen Bewegung der Strahlungsquelle (8) gefolgt wird.

2. System nach Anspruch 1, wobei der Magnet durch einen elektrischen Motor (15) betrieben wird, der ausgeführt ist, um in Bezug auf die Säule (5) fixiert zu sein.

3. System nach Anspruch 2, wobei der elektrische Motor (15) innerhalb der Säule (5) positionierbar ist.

4. System nach einem der vorstehenden Ansprüche, wobei die elektromagnetische Strahlungsquelle (8) toroidal ist.

5. System nach einem der vorstehenden Ansprüche, wobei die elektromagnetische Strahlungsquelle (8) ein UV-C Neonlicht ist.

6. System nach einem der vorstehenden Ansprüche, wobei der Stromversorgung der elektromagnetischen Strahlungsquelle (8) eine Batterie ist, wobei die Batterie an der elektromagnetischen Strahlungsquelle (8) befestigt ist, oder durch ein Kabel getragen wird, wobei das Kabel der Bewegung der elektromagnetischen Strahlungsquelle (8) folgt.

7. System nach einem der vorstehenden Ansprüche, wobei die Aktivierung der Mittel und der elektromagnetischen Strahlungsquelle (8) entweder manuell oder automatisch basierend auf einer vorbestimmten Logik ist.

8. Ein Fahrzeug (1), umfassend mindestens eine Säule (5), die in Bezug auf den Boden (3) des Fahrzeugs (1) befestigt ist, wobei die Säule (5) ausgeführt ist, um Passagieren Halt an der Säule zu ermöglichen, wobei das Fahrzeug (1) ein System (7) zur Desinfektion mindestens einer Säule (5) nach einem der Ansprüche 1 bis 7 umfasst.

9. Fahrzeug nach Anspruch 8, wobei die Säule (5) eine vertikale, horizontale, oder geneigte Säule (5) in Bezug auf dem Boden (3) des Fahrzeugs (1) ist.

10. Verwendung eines Systems (7) nach einem der Ansprüche 1 bis 7 zur Desinfektion einer Säule (5) eines Fahrzeugs des öffentlichen Personenverkehrs.

11. Verwendung eines Systems nach Anspruch 10, wobei die elektromagnetische Strahlungsquelle (8) auf der Säule (5) gleitet, wobei das Gleiten reibungslos in Bezug auf die Säule (5) ist.

## Revendications

1. Système (7) pour assainir une colonne (5) pour un usage public, **caractérisé en ce qu'**il comprend une source de rayonnement électromagnétique assainissant (8) ayant une forme annulaire, ladite source de rayonnement (8) pouvant être positionnée autour de ladite colonne (5) pour coulisser sur celle-ci, ledit système (7) comprenant des moyens d'actionnement (10) configurés pour actionner ladite source de rayonnement (8) automatiquement le long de ladite colonne (5) afin d'assainir une partie préétablie de celle-ci,
dans lequel lesdits moyens d'actionnement (10) comprennent un aimant (11) configuré pour coulisser à l'intérieur de ladite colonne (5), ladite source de rayonnement (8) comprenant une pluralité d'anneaux métalliques (12) fixés à ladite source de rayonnement (8) et espacés uniformément angulairement les uns par rapport aux autres, lesdits anneaux (12) étant magnétisés pour interagir magnétiquement avec ledit aimant (11) de sorte qu'un déplacement de l'aimant (11) à l'intérieur de ladite colonne (5) soit suivi d'un déplacement identique de ladite source de rayonnement (8).

2. Système selon la revendication 1,
dans lequel ledit aimant est actionné par un moteur électrique (15) configuré pour être fixe par rapport à ladite colonne (5).

3. Système selon la revendication 2,
dans lequel ledit moteur électrique (15) peut être positionné à l'intérieur de ladite colonne (5).

4. Système selon l'une des revendications précédentes, dans lequel ladite source de rayonnement électromagnétique (8) est toroïdale.

5. Système selon l'une des revendications précédentes, dans lequel ladite source de rayonnement électromagnétique (8) est une lumière de néon UV-C.

6. Système selon l'une des revendications précédentes, dans lequel l'alimentation de ladite source de rayonnement électromagnétique (8) est une batterie, ladite batterie étant montée sur ladite source de rayonnement électromagnétique (8), ou supportée au moyen d'un câble, ledit câble suivant le déplacement de ladite source de rayonnement électromagnétique (8)

7. Système selon l'une des revendications précédentes, dans lequel l'activation desdits moyens et de ladite source de rayonnement électromagnétique (8) est soit manuelle, soit automatique sur la base d'une logique prédéterminée.

8. Véhicule (1) comprenant au moins une colonne (5) fixe par rapport audit plancher (3) dudit véhicule (1), ladite colonne (5) étant configurée pour permettre aux passagers de se tenir à ladite colonne, ledit véhicule (1) comprenant un système (7) pour assainir au moins une dite colonne (5) selon l'une quelconque des revendications 1 à 7.

9. Véhicule selon la revendication 8, dans lequel ladite colonne (5) est une colonne verticale, horizontale, ou inclinée (5) par rapport au plancher (3) dudit véhicule (1).

10. Utilisation d'un système (7) selon l'une quelconque des revendications 1 à 7 pour assainir une colonne (5) d'un véhicule de transport public.

11. Utilisation d'un système selon la revendication 10, dans laquelle ladite source de rayonnement électromagnétique (8) coulisse sur ladite colonne (5), ledit coulissement étant sans frottement par rapport à ladite colonne (5).
